# EUROPEAN PATENT APPLICATION

(11) **EP 2 270 485 A1**
(43) Date of publication of application: **05.01.2011**
(21) Application number: 09728111.7
(22) Date of filing: 03.04.2009
(51) Int. Cl.: G01N 27/447

(54) **SUPPORT FOR ELECTROPHORESIS INCLUDING HYDROPHOBIC POLYMER MEMBRANE, AND MIGRATION SEPARATION METHOD USING THE SAME**

(30) Priority: 04.04.2008 JP 2008097663; 04.11.2008 JP 2008283318
(71) Applicant: National Institute of Advanced Industrial Science and Technology, Tokyo 100-8921 (JP)
(72) Inventor: KAMEYAMA, Akihiko, Tsukuba-shi Ibaraki 305-8568 (JP); NARIMATSU, Hisashi, Tsukuba-shi Ibaraki 305-8568 (JP); MATSUNO, Yu-ki, Tsukuba-shi Ibaraki 305-8568 (JP)
(74) Representative: Zimmermann & Partner
(86) International application number: PCT/JP2009/056939
(87) International publication number: WO 2009/123303

(57) **Abstract**

There is provided a membrane electrophoresis which renders possible glycan analysis of polysaccharide and glycoprotein by separating protein, glycoprotein or polysaccharide by electrophoresis, followed by carrying out a glycan-releasing treatment of the membrane used in the electrophoresis, or a membrane electrophoresis which renders possible detection of the same by an immunostaining which uses an antibody, wherein a layer containing a hydrophilic polymer is formed on a hydrophobic polymer membrane by coating the hydrophilic polymer on the whole surface of the hydrophobic polymer membrane or by soaking the hydrophobic polymer membrane in a solution of the hydrophilic polymer, which is used as a substrate for electrophoresis.

## Description

### Technical Field

This invention relates to a method for electrophoretically separating proteins, glycoproteins or mucopolysaccharides which are present in a complex protein mixture such as a living body-derived sample, and a substrate for electrophoresis to be used in said method.

### Background of the Invention

Since it is known that a protein which is present in the living body changes its expression level at the time of a disease such as a cancer, its application as a novel diagnostic marker can be expected by grasping a group of molecules changed by the disease. Particularly in the case of a glycoprotein, it is known that, in addition to its quantitative changes at the time of a disease, its glycan structure also changes, so that there is a possibility that it can be used as a further excellent novel diagnostic marker by grasping the glycan structure changed by the disease and a group of molecules including the same. Accordingly, a method for selectively separating and analyzing a group of glycoproteins classified by a certain property has a high possibility of becoming one of the clinical inspections important for the diagnosis and treatment of a disease. For example, a cellulose acetate membrane electrophoresis is used in the separation of serum protein, and a change in its separation pattern and a quantitative change of its specific fraction are used in the diagnosis of diseases as a clinical inspection method.

A mucin-like glycoprotein is a glycoprotein which is present in a mucous liquid or mucous membrane, wherein a mucin type glycan is linked thereto via N-acetylgalactosamine which is O-glycoside-linked to the hydroxyl group of serine or threonine. In addition, the mucin-like glycoprotein is known as a high molecular weight glycoprotein having high sugar content. On the other hand, glycosaminoglycan as a representative of mucopolysaccharides is universally present in every tissue, mainly in connective tissues of animals, and is present in most cases in the form of being added to protein as a proteoglycan. A proteoglycan-type glycoprotein is a glycoprotein having a glycosaminoglycan chain via xylose which is O-glycoside linked to the hydroxyl group of serine. In addition, hyaluronic acid is known as a glycosaminoglycan which is not linked to protein. Hyaluronic acid is present in an extracellular matrix and is also known as a marker molecule of mesothelioma. Since both of these glycoproteins and mucopolysaccharides have been reported to have relation with diseases, these are important as target molecules of novel diagnostic markers. In addition, α-fetoprotein, prostate specific antigen (PSA) and the like which are known as a liver cancer marker or a prostatic cancer marker are glycoproteins contained in a serum. Thus, these serum glycoproteins are also important as the target molecules of novel diagnostic markers.
Conventionally, as a technique for separating these glycoproteins, electrophoresis which can visually grapes their separated state is suitably used, and illustratively, in the case of serum glycoprotein, there may be mentioned an SDS-polyacrylamide gel electrophoresis, a cellulose acetate membrane electrophoresis, a two-dimensional electrophoresis as a combination of a gel isoelectric focusing with an SDS-polyacrylamide gel electrophoresis, and the like, In addition, in the case of a mucin-like glycoprotein or a proteoglycan-type glycoprotein, there may be mentioned an electrophoresis which uses an agarose gel or an agarose/polyacrylamide mixed gel (cf. Non-patent References 1 and 2), a cellulose acetate membrane electrophoresis (cf. Non-patent Reference 3) and the like.
In addition, in order to analyze glycans of the glycoprotein separated by electrophoresis, a conventionally known transferring method can be employed. That is, there my be used a method in which a glycoprotein separated by electrophoresis is transferred from the gel onto a polyvinylidene difluoride (PVDF) membrane, and then glycans are cut out by a β-elimination reaction through an enzyme reaction or an alkali treatment on said membrane and the thus cut out glycans are analyzed by a mass spectrometer (cf. Non-patent Reference 2).

On the other hand, regarding the method for separating and analyzing protein by electrophoresis, Patent Reference 1 proposes a method in which lipoprotein contained in a specimen is separated by electrophoresis using a cellulose acetate membrane containing a hydrophilic polymer such as a dextran having a molecular weight of from 40,000 to 2,000,000, and a detection of a disease caused by abnormal lipid metabolism by confirming existing mode of subclasses of the separated lipoprotein VLDL, LDL and HDL.

### Related Art References

### Patent References

Patent Reference I : JP-A-2007-248131
Non-Patent References

Non-patent Reference 1: Spurr-Michaud S et al. Assay of mucins in human tear fluid. Exp. Eye Res. 2007, 85, 939-950
Non-patent Reference 2: Thomsson KA, Schulz BL, Packer NH, Karlsson NG. MUC5B glycosylation in human saliva reflects blood group and secretor status. Glycobiology 2005,15, 791-804.
Non-patent Reference 3: Yasueda SI, Yamakawa K, Nakanishi Y, Kinoshita M, Kakehi K. Decreased mucin concentrations in tear fluids of contact lens wearers. J. Pharm. Biomed. Anal. 2005, 39, 187-195.

### Outline of the Invention

### Problems That the Invention is to Solve

However, since the conventionally known SDS-polyacrylamide gel electrophoresis, two-dimensional electrophoresis as a combination of a gel isoelectric focusing with an SDS-polyacrylamide gel electrophoresis, or the electrophoresis described in Non-patent References 1 and 2 which uses an agarose gel or an agarose/polyacrylamide mixed gel requires a step for transferring the glycoproteins onto a PVDF membrane after their separation, they lack in the throughput property so that their application as a diagnostic method in the clinical field is difficult to attain. Also, lowering of the quantitative property due to transferring efficiency, and the like, cause problems.
In addition, the method described in Patent Reference 1 is concerned in lipoproteins and does not describe on glycoproteins or mucopolysaccharides, and the lipoproteins after electrophoresis are transferred onto a PVDF membrane, nitrocellulose membrane or the like and then the lipoproteins transferred on the membrane are confirmed also in this method, so that it poses the same problems of the case of the methods described in Non-patent References 1 and 2.

On the other hand, the cellulose acetate membrane electrophoresis described in Non-patent Reference 3 renders possible separation of mucin-like glycoprotein and proteoglycan-type glycoprotein on the membrane so that omission of the transferring step can be expected. However, since the membrane-derived cellulose derivative formed by the β-elimination reaction obstructs the subsequent glycan analysis, information on the glycan structures of mucin-like glycoprotein and proteoglycan-type glycoprotein cannot be obtained by this method.

As described in the above, there has been no membrane electrophoresis method which renders possible glycan analysis of a glycoprotein or mucopolysaccharide by separating the glycoprotein or mucopolysaccharide by an electrophoresis method and then carrying out a glycan-releasing treatment with the membrane used in the electrophoresis. In addition, there has been no membrane electrophoresis method which renders possible the detection by an immunostaining making use of an antibody with the membrane used in the electrophoresis.
The invention has been made by taking the above-mentioned situations into consideration, and it aims at providing a convenient method for separating and analyzing a protein or mucopolysaccharide, which can be applied to a marker searching and a diagnosis of diseases in the clinical field.

### Means for Solving the Problems

As described in the above, when a PVDF membrane is used, glycans of a glycoprotein can be cut out by a β-elimination reaction through an alkali treatment on the membrane, so that it is possible to analyze the cut out glycans using a Mass spectrometer. In addition, it is also possible to detect a specific protein by specifically staining it using an antibody.
Accordingly, based on an assumption that the problems can be solved when a PVDF membrane can be used as the substrate of electrophoresis, the present inventors have conducted intensive studies on a method for separating a protein or mucopolysaccharide using a PVDF membrane as the substrate of electrophoresis and found as a result that a protein or mucopolysaccharide can be migrated and separated by coating whole surfaces of the PVDF- membrane with a solution of a hydrophilic polymer and making use the membrane as the substrate for electrophoresis.
Also, in addition to the PVDF membrane, when separation of a protein, glycoprotein or mucopolysaccharide was examined using hydrophobic polymer membranes for protein immobilization which have not been used in electrophoresis, it was found that the protein, glycoprotein or mucopolysaccharide can be migrated and separated by electrophoresis which uses a membrane prepared by coating a polytetrafluoroethylene membrane (PTFE) or nylon membrane (Nylon) with a solution of a hydrophilic polymer.

The invention has been accomplished based on these findings, which is as follows.
[1] A substrate for electrophoresis, which comprises a hydrophilic polymer layer on a hydrophobic polymer membrane.
[2] The substrate for electrophoresis according to [1], wherein the hydrophilic polymer is at least one kind selected from polyvinyl pyrrolidone, polyvinyl alcohol, polyethylene glycol, polyethylene oxide and poly-2-hydroxyethyl methacrylate.
[3] The substrate for electrophoresis according to [1] or [2], wherein the hydrophobic polymer membrane is at least one kind selected from polyvinylidene difluoride, nylon and polytetrafluoroethylene.
[4] The substrate for electrophoresis according to any one of [1] to [3], which is used for separation of a protein or a mucopolysaccharide.
[5] A method for separating a protein, wherein the protein is separated by electrophoresis using the substrate for electrophoresis according to any one of [1] to [3].
[6] The method for separating a protein according to [5], wherein the protein is a mucin-like glycoprotein.
[7] The method for separating a protein according to [5], wherein the protein is a proteoglycan-type glycoprotein.
[8] A method for separating a mucopolysaccharide, wherein the mucopolysaccharide is separated by electrophoresis using the substrate for electrophoresis according to [1] to [3].
[9] A kit for separation of a protein or a mucopolysaccharide, which is used in separating the protein or mucopolysaccharide by electrophoresis, and comprises the substrate for electrophoresis according to [1] to [3].
[10] A method for separating and detecting a protein, wherein the protein contained in a specimen is separated by the separation method according to [5] to [7], and then the separated protein on a membrane is reacted with an anti-protein antibody as a primary antibody, followed by reacting with an enzyme-labeled secondary antibody, followed by detecting the protein using a color developing agent.
[11] A method for separating and detecting a glycoprotein ar a mucopolysaccharide, wherein the glycoprotein or mucopolysaccharide contained in a specimen is separated by the separation method according to [6] to [8], and then a glycan of the separated glycoprotein or mucopolysaccharide on a membrane is oxidized with periodic acid, followed by reacting with a pigment-labeled amine derivative to detect the glycoprotein or mucopolysaccharide.
[12] A method for releasing a glycan from a glycoprotein or mucopolysaccharide separated by the method for separating a glycoprotein or mucopolysaccharide according to [6] to [8], wherein a glycan-releasing treatment is carried out on the substrate used in electrophoresis.
[13] A kit for releasing a glycan, which is used in releasing the glycan from a glycoprotein or mucopolysaccharide separated by electrophoresis, by carrying out a glycan-releasing treatment on the substrate used in electrophoresis, and comprises the substrate for electrophoresis according to [1] to [3].
[14] A method for analyzing a glycan structure, wherein the glycan structure in a glycoprotein or mucopolysaccharide separated by the electrophoresis is analyzed using the glycan released by the releasing method according to [13],
[15] A kit for analyzing a glycan structure, which is used in analyzing the glycan structure in a glycoprotein or mucopolysaccharide separated by the electrophoresis, by releasing a glycan from a glycoprotein or mucopolysaccharide separated by electrophoresis, through a glycan-releasing treatment on the substrate used in the electrophoresis, to use said released glycan, and compirses the substrate for electrophoresis according to [1] to [3].

### Advantage of the Invention

According to the invention, since protein, glycoprotein or mucopolysaccharide can be migrated and separated on a polymer having high hydrophobicity which is used in the protein immobilization, the transferring step can be omitted and throughput of analysis is improved in comparison with the conventional methods. In addition, since the invention is quick and convenient and also renders possible antibody staining and glycan analysis, its application to a diagnostic kit can be expected making use of disease-related glycans on protein, glycoprotein or polysaccharide as the index.

### Brief Description of the Drawings

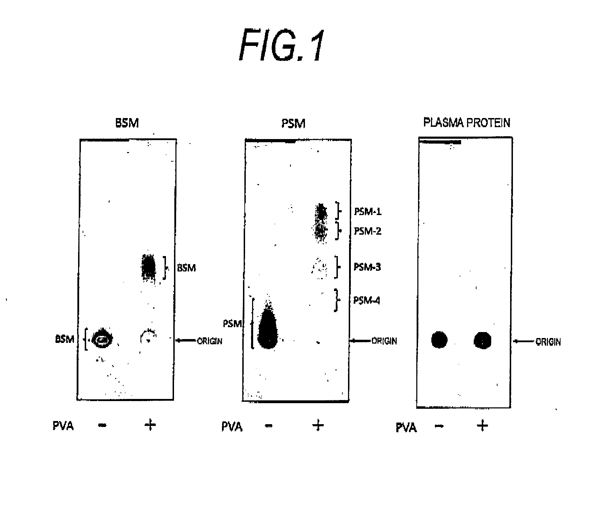

[Fig.1] A view showing a result of carrying out electrophoresis of BSM, PSM and blood plasma-derived protein on a PVA-uncoated PVDF membrane and a PVA-coated PVDF membrane, respectively.
[Fig. 2] A view showing a result of releasing glycans from BSM separated by a PVA-coated PVDF membrane and analyzing them by mass spectrometry.
[Fig. 3] A view showing a result of releasing glycans from PSM separated by a PVA-coated PVDF membrane and analyzing them by mass spectrometry.
[Fig. 4] A view showing a result of carrying out electrophoresis of PSM before its treatment with chondroitinase ABC and PSM after the treatment, using a PVA-coated PVDF membrane.
[Fig.5] A view showing a result of carrying out electrophoresis of mucin in human saliva using a PVA-coated PVDF membrane and then staining it with Alcian Blue, Pro-Q Emerald or by immunostaining.
[Fig. 6] A view showing a result of carrying out electrophoresis of BSM, PSM and plasma-derived protein using a PVDF membrane coated with polyvinyl pyrrolidone, polyethylene glycol or poly 2-hydroxyethyl methacrylate, respectively.
[Fig. 7] A view showing a result of carrying out electrophoresis of plasma-derived protein using a cellulose acetate membrane and a PVA-coated PVDF membrane.
[Fig. 8] A view showing a result of carrying out electrophoresis of plasma-derived protein using a PVA-coated PVDF membrane, a polytetrafluoroethylene membrane (PTFE) and a nylon membrane (Nylon).
[Fig. 9] A view showing a result of carrying out electrophoresis of human plasma protein and staining it with anti-human haptoglobin.

### Mode for Carrying Out the Invention

The method of the invention for separating and analyzing protein, glycoprotein or mucopolysaccharide is based on the electrophoresis which uses a hydrophobic polymer membrane on which a hydrophilic polymer layer is formed.
The hydrophobic polymer membrane to be used in the invention is a polymer membrane having a property of strongly binding and keeping protein by a hydrophobic interaction, and there may be particularly mentioned a PVDF membrane, polytetrafluoroethylene membrane (PTFE) and a nylon membrane (Nylon), of which PVDF membrane is most desirable from the viewpoint of separation ability.
As described in Patent Reference 1 and Non-patent Reference 2, these hydrophobic polymer membranes are originally used mainly for protein immobilization in order to carry out identification of a glycoprotein, an antigenic protein and the like on the membrane by adding a purified protein solution onto the membrane or transferring protein separated by electrophoresis onto the membrane.

According to the invention, it becomes possible to separate protein, glycoprotein or mucopolysaccharide by the use of a substrate for electrophoresis in which a hydrophilic polymer layer is formed on the aforementioned hydrophobic polymer membrane.
In addition, since the separated protein, glycoprotein or mucopolysaccharide is present on the hydrophobic polymer membrane having the hydrophilic polymer layer, when β-elimination reaction is carried out on the membrane as such using a non-cellulose-based material in the hydrophobic polymer, unlike the case of the conventional cellulose membrane, the membrane-derived cellulose derivative caused by the β-elimination reaction does not obstruct the subsequent glycan analysis. That is, according to the method of the invention for analyzing protein, glycoprotein or mucopolysaccharide, it becomes possible to analyze protein, glycoprotein or mucopolysaccharide by a convenient method which does not require the transferring step.

Those which are conventionally used as the membranes for transferring protein separated by electrophoresis can be used as such in the hydrophobic polymer membrane to be used in the invention.
As the method for forming a hydrophilic polymer layer on said hydrophobic polymer membrane, illustratively, there may be mentioned a method in which the hydrophobic polymer membrane is soaked in a hydrophilic polymer solution, a method in which a hydrophilic polymer is coated on the hydrophobic polymer membrane, a method in which a hydrophilic polymer film is laminated thereon, and the like.

According to the invention, the polymer which forms a hydrophilic layer is a polymer that contains at least one hetero atom such as oxygen or nitrogen in the polymer constituting unit, which is a polymer having affinity with water of 60° or less in water contact angle. As examples, there may be mentioned polyvinyl pyrrolidone, polyvinyl alcohol, polyacrylamide, polyethylene glycol, polyethylene oxide and the like, of which polyvinyl pyrrolidone, polyvinyl alcohol and polyethylene glycol are particularly suitable from the view point of separation ability and most suitable is polyvinyl alcohol.
In this connection, in view of the object of the invention, it is a matter of course that polysaccharide-based polymers such as dextrin are not included in the hydrophilic polymer to be used in the invention.
In addition, it is desirable that molecular weight of the hydrophilic polymer to be used in the invention is from 1,000 to 4,000,000,

As the apparatus to be used in the invention, a conventional cellulose acetate membrane electrophoresis apparatus can be used as such, and illustratively, EPC105AA type cellulose acetate membrane electrophoresis apparatus (manufactured by ADVANTEC) and the like can be used.
The electrophoresis which uses the aforementioned substrate for electrophoresis of the invention is carried out in accordance with the techniques described in "Tanpakushitsu to Koso no Also Jikken-ho (Fundamental Experimentation Methods of proteins and Enzymes)" (published by Nankodo), "Shin Seikagaku Jikken Koza 3 - Toshitsu II (New Biochemistry Experimentation Course 3 - Sugar II)" (published by Tokyo Kagaku Dojin) and the like.
That is, a veronal buffer, a tris buffer, a pyridine-formate buffer and the like are used as the buffer for electrophoresis. For the separation of protein and glycoprotein, it is desirable to use the veronal buffer or tris buffer at around neutral pH. When further distinct separation is carried out on mucin-like protein and proteoglycan-type glycoprotein, the pyridine-formate buffer (from pH 3.0 to pH 5.0) is suitable. In addition, it is considered that coating amount of a specimen is generally from 0.8 to 2.4 µl per 1 spot or per 1 cm in width, and as the current applying condition, it is desirable to apply a current of approximately from 0.5 to 1.5 mA per 1 cm in width. Also, temperature of the substrate during the electrophoresis is generally set to a constant level within a range of from 10 to 20°C.

As described in the foregoing, the aforementioned substrate for electrophoresis of the invention can undergo a glycan-releasing treatment as such without further transfer onto other membrane, and as the glycan-releasing treatment according to the invention, there may be used a release reaction which uses an enzyme and the β-elimination reaction described in the Non-patent Reference 2.
Illustratively, in the case of the release reaction by an enzyme, a solution containing glycan cutting enzyme such as N-glycanase is added to a piece of membrane prepared by cutting out a spot or band containing glycoprotein, followed by carrying out the reaction overnight at 37°C. After the reaction, the released glycan is obtained by removing the salts. In the case of the β-elimination reaction, an aqueous solution containing an alkali such as sodium hydroxide and a reducing agent such as sodium borohydride is added to a piece of membrane prepared by cutting out a spot or band containing glycoprotein, followed by carrying out the reaction at 45°C for 16 hours. After the reaction, the released glycan is obtained by neutralizing with an acid and removing the salts.

Further, as the method for analyzing the glycan structure of the thus released glycan, there are high performance liquid chromatography (HPLC), nuclear magnetic resonance spectrometry (NMR), mass spectrometry and the like. Particularly, mass spectrometry is desirable in view of sensitivity, accuracy and convenience.
In addition, as described in the foregoing, the aforementioned substrate for electrophoresis of the invention can detect a specific protein by an antibody staining as such without further transfer onto other membrane.

### EXAMPLES

The following describes the invention further illustratively based on example, though the invention is not limited by these examples.

### <Example 1>

In this Example, electrophoresis was carried out using a bovine submandibular gland mucin (BSM), a pig stomach mucin (PSM) and a plasma-derived protein.

### (Preparation of PVDF membrane coated with hydrophilic polymer)

A commercially available PVDF membrane (Immobilon-P, mfd. by Millipore) was cut out into an appropriate size and soaked in methanol for several minutes. Subsequently, said PVDF membrane was taken out from methanol and soaked for 30 minutes in a buffer for electrophoresis (0.1 M pyridine-formate buffer, pH 4,0) containing 0.25% polyvinyl alcohol.
Just before spotting a sample, the membrane was taken out from the aforementioned buffer for electrophoresis and used after lightly wiping off excess solution adhered to the membrane using a filter paper.

### (Pretreatment of analyzing samples)

Each of BSM (50 µg), PSM (100 µg) and plasma-derived protein (prepared by desalting and freeze-drying blood plasma, about 50 µg) were dissolved in 0.1 M Tris-HCl buffer (pH 8.6, 10 µl) containing 20 mM dithiothreitol and 8 M urea and allowed to undergo the reaction at 100°C for 20 minutes. After spontaneously cooling this at room temperature, 250 mM iodoacetamide aqueous solution (1 µl, final concentration 25 mM) was added thereto and allowed to undergo the reaction in the dark at room temperature for 1 hour.
After the reaction, a portion of the sample solution (1 µl) was spotted on the aforementioned PVDF membrane coated with PVA and subjected to electrophoresis.

### (Electrophoresis)

A cellulose acetate membrane electrophoresis apparatus (Model EPC105AA, manufactured by ADVANTEC) was used as the electrophoresis vessel. The current applying condition was set to 1,0 mA per 1 cm in membrane width, and the electrophoresis was carried out for 30 minutes.

### (Staining method)

A 0.1 % Alcian Blue 8GX solution which was dissolved in 0.1% acetic acid was used in the BSM and PSM staining. Also, a 0.008% Direct Blue 71 solution which was dissolved in 40% ethanol/10% acetic acid aqueous solution was used in the staining of plasma-derived protein.

### (Results of electrophoresis)

Results of electrophoresis are shown in Fig, 1. These are results of BSM, PSM and plasma-derived protein in order from the left side, and in each of them, the left side shows a result of using a PVA-uncoated substrate (Comparative Example) and the right side shows a result of using the substrate of the invention.
As is evident from Fig, 1, BSM and plasma-derived protein were hardly migrated from the origin when their electrophoresis was carried out using the PVDF membrane uncoated with PVA (Comparative Example). Also, though PSM was slightly migrated, tailing of its spot was observed so that it does not result in complete separation.
On the other hand, in the case of the use of the PVA-coated PVDF membrane (the invention), BSA was migrated as a single component and PSM was migrated and separated as 4 kinds of components. However, the plasma-derived protein was not migrated from the origin even in the case of the PVA coating.
Based on this, it was found that the mucin-like glycoprotein is selectively migrated and separated by the use of the PVA-coated PVDF membrane of the invention as the substrate for electrophoresis.

### (Release of glycan)

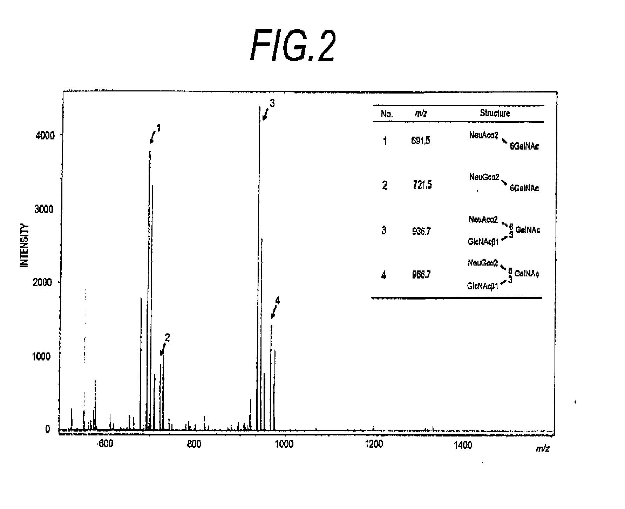

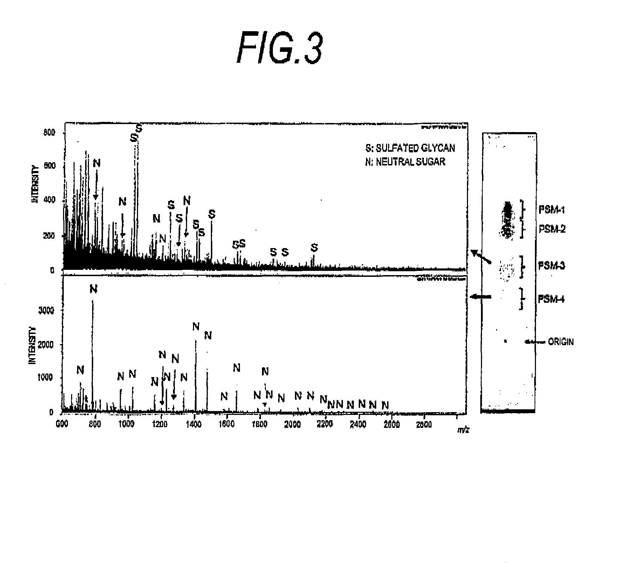

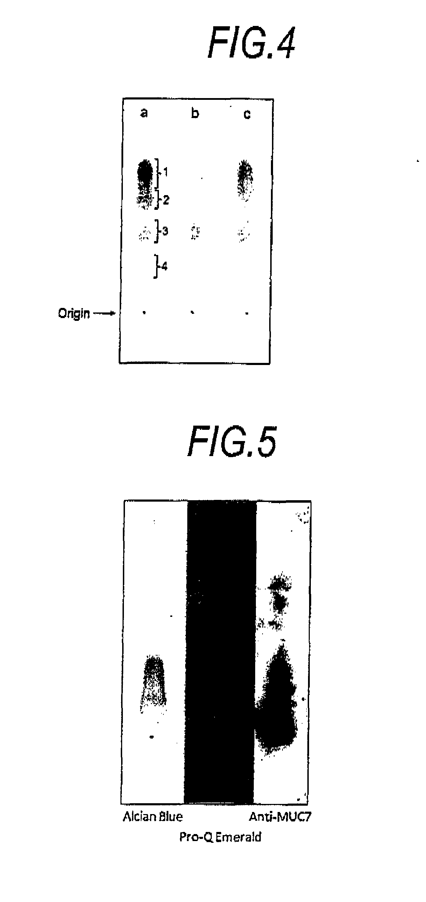

Next, each of the spots of BSM and PSM migrated and separated by the aforementioned method of the invention was cut out together with the membrane part and subjected to the β-elimination reaction by an alkali, thereby releasing glycans from the core protein of mucin. Complete methylation derivatives of the thus obtained glycans were measured using a mass spectrometer, and the results are shown in Figs. 2 and 3,
As shown in Fig. 2, 4 kinds of glycan peaks were detected as the main components in the case of BSM, and their mass numbers coincided with the reported mass numbers of the main glycan structures in BSM.
Thus, it was found that the invention can be used also in the glycan analysts after separation of the mucin-like glycoprotein.
In addition, as shown in Fig. 3, in the case of the PSM separated as 4 kinds of components, glycan peaks were detected from the components 3 and 4. It was found that the component 3 firstly migrated among these two components was high in the ratio of sulfated glycan and the component 4 lately migrated was high in the ratio of neutral sugar. Thus, since the respective components in PSM separated by the invention showed different glycan structure patterns, it was found that the invention can separate a group of molecules having different glycan structures, which are present in plural numbers in the mucin-like glycoprotein,

### (Migration of samples treated with degradation enzyme)

Fig. 4 shows a result or carrying out electrophoresis of PSM after its treatment with chondroitinase ABC and hyaluronidase, using a PVA-coated PVDF membrane. In this connection, a result of untreated PSM is shown in Fig. 4a as a reference.
As shown in Fig. 4b, in the case of carrying out electrophoresis of a sample which was treated in advance with chondroitinase ABC as a degradation enzyme of chondroitin sulfates, among the respective components of PSM, spot of the most quickly migrated component 1 almost disappeared. Accordingly, it was suggested that these components are chondroitin sulfate type proteoglycan contaminated in PSM.
In addition, as shown in Fig, 4c, in the case of carrying out electrophoresis of a sample which was treated in advance with hyaluronidase as a degradation enzyme of hyaluronic acid, among the respective components in PSM, spot of the most quickly migrated component 2 almost disappeared. Accordingly, it was suggested that these components are hyaluronic acid contaminated in BSM.
Based on this, it was found that the invention can be applied to the analysis of not only the mucin-like glycoproteins but also the mucopolysaccharides such as glycosaminoglycan and the proteoglycan-type glycoproteins.

### <Example 2>

In this Example, separation of mucin in human saliva by the invention and staining of glycoprotein making use of periodate oxidation of glycan and immunostaining by an antibody were carried out,

### (Preparation of PVDF membrane coated with PVA)

It was prepared by the same procedure of Example 1.

### (Pretreatment of analyzed sample)

About 500 µl of human saliva was centrifuged at 10000 g for 5 minutes, and the supernatant was desalted and concentrated using an ultrafiltration membrane (100 kDa cutoff) and then freeze-dried to be used as a crude mucin fraction. The freeze-dried sample was dissolved in 0.1 M Tris-HCl buffer (pH 8.6, 20 µl) containing 20 mM dithiothreitol and 8 M urea and allowed to undergo the reaction at 100°C for 20 minutes. After spontaneously cooling this at room temperature, 250 mM iodoacetamide aqueous solution (2 µl, final concentration 25 mM) was added thereto and allowed to undergo the reaction in the dark at room temperature for 1 hour.
After the reaction, the sample solution was diluted two times with 0.1 M Tris-HCl buffer (pH 8.6) containing 8 M urea, and a portion (1 µl) thereof was spotted on the aforementioned PVDF membrane coated with PVA and subjected to electrophoresis.

### (Electrophoresis)

It was carried out by the same procedure of Example 1,

### (Staining method)

Staining with Alcian Blue was carried out in the same manner as in Example 1. Pro-Q Emerald (manufactured by Molecular Probes) was used in the staining of glycoprotein which makes use of the periodate oxidation of glycan. Immunostaining with antibody was carried out in accordance with a conventionally known method, after shaking the membrane after electrophoresis for 30 minutes in 5% acetic acid/methanol to effect immobilization of glycoprotein onto the membrane. That is, the membrane after immobilization was washed with PBS-T by shaking, followed by shaking in PBS-T containing 1% BSA to effect blocking. The membrane was washed with PBS-T by shaking, followed by soaking in a solution prepared by diluting anti-MUC 7 goat IgG 200 times with PBS-T, followed by shaking for 3 hours. The membrane was washed with PBS-T by shaking, followed by soaking in a solution prepared by diluting horseradish peroxidase-labeled anti-goat IgG 1000 times with PBS-T, followed by shaking for 1 hour. After washing with PBS-T by shaking, detection was carried out by the ECL method.

### (Results of electrophoresis and staining)

A result of electrophoresis of mucin in human saliva and a result of staining by respective staining methods are shown in Fig. 5.
When mucin in human saliva was subjected to the electrophoresis of the invention and then stained with Alcian Blue, it was observed as a broad band as shown in the drawing (left side of the drawing). On the other hand, when stained with Pro-Q Emerald, a staining pattern similar to the case of the staining with Alcian Blue was obtained (central of the drawing). In the case of the immunostaining with anti-MUC 7 antibody, significant nonspecific adsorption of the antibody to the protein at the origin was found, but the site stained by Alcian Blue and Pro-Q Emerald was mainly stained (right side of the drawing). In addition, a quickly migrating component was slightly detected only at the time of immunostaining.
Based on the above results, it was found that the glycoprotein separated by the electrophoresis which uses the PVDF membrane of the invention which is coated with a hydrophilic polymer can also be detected by the staining of glycoprotein which makes use of the periodate oxidation, af glycan and the immunostaining with antibody.

### <Example 3>

### (Examination by other hydrophilic polymers)

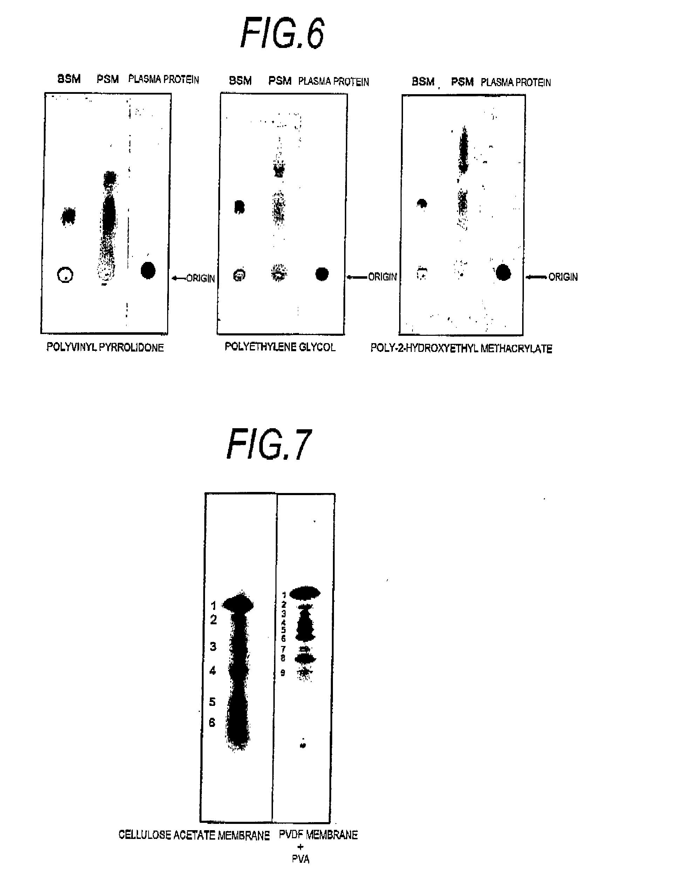

Fig. 6 shows a result of carrying out electrophoresis of BSM, PSM and plasma-derived protein by using the PVDF membrane which was coated in the same procedure of Example 1 using hydrophilic polymers other than PVA. There are shown results of using PVDF membranes coated with polyvinyl pyrrolidone, polyethylene glycol and poly-2-hydroxyethyl methacrylate in order from the left side.
As is evident from Fig. 6, it was found that when the coating is carried out using hydrophilic polymers other than PVA, the effects equivalent to the case of PVA can also be obtained and glycosaminoglycan and mucin-like glycoprotein are selectively migrated and separated.

### <Example 4>

### (Separation of plasma protein)

The invention can also be used for the separation of a complex protein mixture such as plasma protein by changing the kind of the buffer to be used in electrophoresis. Electrophoresis of plasma protein was effected by the invention using 0.06 M barbital sodium buffer (pH 8.6) as the buffer for electrophoresis. In addition, cellulose acetate membrane electrophoresis was carried out as a control. In Fig. 7, a result of carrying out the electrophoresis using cellulose acetate membrane and a result of the electrophoresis by the invention are shown and compared. As shown in Fig. 7, the plasma protein was separated into 6 fractions by the cellulose acetate membrane electrophoresis, while the plasma protein was separated into 9 fractions by the invention. Thus, it was found that the invention has high resolution.

### <Example 5>

### (Examination by other hydrophobic polymer membranes)

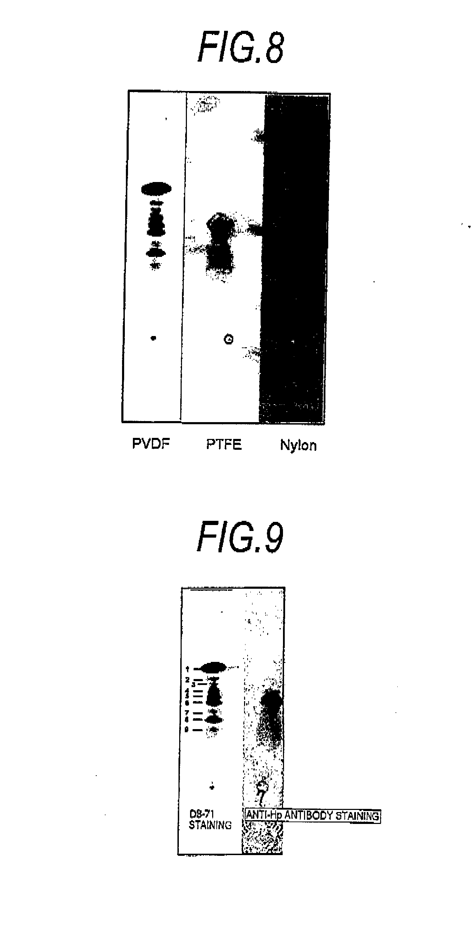

Hydrophobic polymer membranes other than the PVDF membrane were also coated with PVA in the same procedure of Example 1, and Fig. 8 shows the examples that plasma protein was separated using these membranes. It shows a result of using PVDF membrane, polytetrafluoroethylene membrane (PTFE) and nylon membrane (Nylon) in order from the left side. Though the migration pattern varied depending on the used hydrophobic polymer membranes, separation by the electrophoresis was observed. Thus, Fig. 8 shows that the hydrophobic polymer membrane to be used in the invention is not limited to the PVDF membrane.

### <Example 6>

### (Antibody staining of haptoglobin in plasma)

Electrophoresis of plasma protein was carried out by the invention, in the same procedure of Example 4. The immunostaining by a haptoglobin antibody was carried out in accordance with a conventionally known method after shaking the membrane which had been subjected to electrophoresis for 15 minutes in acetone, followed by immobilizing plasma protein onto the membrane. That is, the membrane after immobilization was shaken in PBS containing 1 % BSA for 1 hour to effect blocking. The membrane was washed with PBS-T (0.05% tween) for 5 minutes, and this washing operation was repeated 3 times in total. After the shaking, this was soaked in a solution prepared by diluting a rabbit anti-human haptoglobin antibody (IgG, 1 mg/ml) 2000 times with PBS-T, followed by shaking for 1 hour. Thereafter, the membrane was washed with PBS-T (0.05% tween) for 5 minutes and, after repeating this washing operation 3 times in total, it was soaked in a solution prepared by diluting a horseradish peroxidase-labeled goat anti-rabbit IgG antibody 2000 times with PBS-T, followed by shaking for 1 hour. Thereafter, the membrane was washed with PBS-T (0.05% tween) for 5 minutes and this washing operation was repeated 3 times in total, followed by carrying out the detection using a Konica immunostain kit (Immunostain HRP-1000, mfd. by KONICA MINQLTA).

### (Results of staining)

A result of carrying out electrophoresis of the human plasma protein and a result of staining it with anti-human haptoglobin are shown in Fig. 9.
As a result of carrying out electrophoresis of human plasma protein by the invention and subsequent staining with DB-71, it was observed as 9 bands as shown in Fig. 9 (Fig. 9-left). On the other hand, when stained with the anti-haptoglobin antibody, a staining pattern of single band was obtained (Fig. 9-right). It is considered that haptoglobin is present in the band 4, based on the similarity with the data on plasma protein by the conventionally used cellulose acetate membrane electrophoresis (Hyojun Rinsho Kensa Igaku (Standard Clinical Inspection Medicine) second edition, published by Igaku Shin). Since the band stained with anti-haptoglobin antibody coincided with the band 4, it is considered that the haptoglobin in plasma was specifically stained by this technique.

Based on the above results, it was found that the protein separated by the electrophoresis which uses a PVDF membrane coated with the hydrophilic polymer of the invention can be specifically detected by antibody immunostaining without transferring onto a hydrophobic membrane for protein immobilization.

### Industrial Applicability

When a substrate for electrophoresis including a hydrophilic polymer layer on the hydrophobic polymer membrane of the invention is used, protein, glycoprotein or mucopolysaccharide can be separated selectively and distinctively, and analysis of the glycan structure presenting in the glycoprotein or mucopolysaccharide becomes possible by allowing the glycoprotein or mucopolysaccharide separated on said substrate to undergo the glycan-releasing reaction without modification. In addition, it is also possible to detect the protein, glycoprotein or mucopolysaccharide separated on said substrate, by an immunostaining which uses a specific antibody or a glycoprotein staining method that makes use of periodate oxidation of glycan. Based on these, an application as an inspection kit to be used in the detection of diseases is expected in the invention.

## Claims

1. A substrate for electrophoresis, which comprises a hydrophilic polymer layer on a hydrophobic polymer membrane.

2. The substrate for electrophoresis according to claim 1, wherein the hydrophilic polymer is at least one kind selected from polyvinyl pyrrolidone, polyvinyl alcohol, polyethylene glycol, polyethylene oxide and poly-2-hydroxyethyl methacrylate.

3. The substrate for electrophoresis according to claim 1 or 2, wherein the hydrophobic polymer membrane is at least one kind selected from polyvinylidene difluoride, nylon and polytetrafluoroethylene.

4. The substrate for electrophoresis according to any one of claims 1 to 3, which is used for separation of a protein or a mucopolysaccharide,

5. A method for separating a protein, wherein the protein is separated by electrophoresis using the substrate for electrophoresis according to any one of claims 1 to 3.

6. The method for separating a protein according to claim 5, wherein the protein is a mucin-like glycoprotein.

7. The method for separating a protein according to claim 5, wherein the protein is a proteoglycan-type glycoprotein.

8. A method for separating a mucopolysaccharide, wherein the mucopolysaccharide is separated by electrophoresis using the substrate for electrophoresis according to any one of claims 1 to 3.

9. A kit for separation of a protein or a mucopolysaccharide, which is used in separating the protein or mucopolysaccharide by electrophoresis, and comprises the substrate for electrophoresis according to any one of claims 1 to 3.

10. A method for separating and detecting a protein, wherein the protein contained in a specimen is separated by the separation method according to any one of claims 5 to 7, and then the separated protein on a membrane is reacted with an anti-protein antibody as a primary antibody, followed by reacting with an enzyme-labeled secondary antibody, followed by detecting the protein using a color developing agent.

11. A method for separating and detecting a glycoprotein or a mucopolysaccharide, wherein the glycoprotein or mucopolysaccharide contained in a specimen is separated by the separation method according to any one of claims 6 to 8, and then a glycan of the separated glycoprotein or mucopolysaccharide on a membrane is oxidized with periodic acid, followed by reacting with a pigment-labeled amine derivative to detect the glycoprotein or mucopolysaccharide.

12. A method for releasing a glycan from a glycoprotein or mucopolysaccharide separated by the method for separating a glycoprotein or mucopolysaccharide according to any one of claims 6 to 8, wherein a glycan-releasing treatment is carried out on the substrate used in electrophoresis.

13. A kit for releasing a glycan, which is used in releasing the glycan from a glycoprotein or mucopolysaccharide separated by electrophoresis, by carrying out a glycan-releasing treatment on the substrate used in electrophoresis, and comprises the substrate for electrophoresis according to any one of claims 1 to 3.

14. A method for analyzing a glycan structure, wherein the glycan structure in a glycoprotein or mucopolysaccharide separated by the electrophoresis is analyzed using the glycan released by the releasing method according to claim 13.

15. A kit for analyzing a glycan structure, which is used in analyzing the glycan structure in a glycoprotein or mucopolysaccharide separated by the electrophoresis, by releasing a glycan from a glycoprotein or mucopolysaccharide separated by electrophoresis, through a glycan-releasing treatment on the substrate used in the electrophoresis, to use said released glycan, and compirses the substrate for electrophoresis according to any one of claims 1 to 3.
